# EUROPEAN PATENT APPLICATION

(11) **EP 3 771 707 A1**
(43) Date of publication of application: **03.02.2021**
(21) Application number: 19189236.3
(22) Date of filing: 30.07.2019
(51) Int. Cl.: C07B 37/04, C07B 43/04, C07D 213/127, C07D 217/24, C07D 409/04, C07F 15/04

(54) **AIR-STABLE NI(0)-OLEFIN COMPLEX AND ITS USE AS CATALYST AND PRECATALYST**

(71) Applicant: Studiengesellschaft Kohle mbH, 45470 Mülheim (DE)
(72) Inventor: CORNELLA, Josep, 45468 Mülheim an der Ruhr (DE); NATTMANN, Lukas, 45468 Mülheim an der Ruhr (DE)

(57) **Abstract**

The present invention relates to an air stable, binary Ni(0)-olefin complex and its use in organic catalysis.

## Description

The present invention relates to an air stable, binary Ni(0)-olefin complex and its use in organic catalysis.

In recent years, nickel (Ni) catalysis has become a growing and empowering area of research due to novel disconnections and reactivity modes towards organic synthesis. In these endeavors, Ni(0)-olefin complexes have become a powerful source of Ni(0) due to their high affinity for ligand exchange. For example, Ni(COD)₂ (bis(cyclooctadiene)Ni(0)) has become the cornerstone Ni(0) source for exploring new catalytic reactivity.

However, binary Ni(0) complexes bearing solely olefins as ligands suffer from great instability and fast decomposition when exposed to air, thus restricting its manipulation to Schlenk techniques or glovebox under inert atmosphere.

In 1960, DE 1 191 375 AS disclosed the synthesis of the first binary metal-olefin complexes as a reaction of olefins and Ni salts. Since this disclosure, Ni(0)-olefin compounds, and specifically Ni(COD)₂, have served as precatalysts to unfold a variety of transformations that impacted all levels of the chemical sciences. Moreover, Ni(COD)₂ and all-*trans*-Ni(CDT) have served as catalyst for various important industrial processes occurring at multi-ton scale, namely polymerization and cyclotrimerization of olefinic compounds.

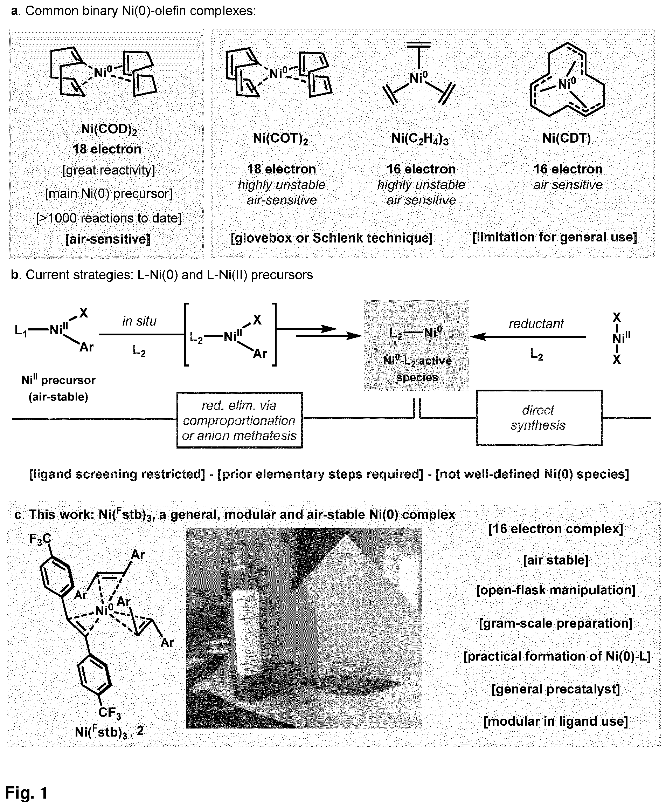

However, in the context of homogeneous catalysis, Ni(COD)₂ has become the main, if not the only, Ni(0) source utilized for reaction discovery (Fig. 1a). Indeed, Ni(COD)₂ is commercially available due to its remarkable stability under inert atmosphere at low-temperatures. The lability of the olefinic ligands in Ni(COD)₂ when competing with more nucleophilic counterparts such as phosphines, diamines or carbenes has placed this compound at the vanguard of reaction discovery, thus reigning sovereign in a plethora of catalytic transformations. However, despite its significant properties, the use of Ni(COD)₂ is linked to its high instability and immediate decomposition upon exposure to air, resulting in tedious manipulations and requiring the use of glovebox or Schlenk techniques. Alternative binary Ni(0)-olefin complexes are restricted to Ni(CDT) (*cis or trans*), Ni(COT)₂ or Ni(C₂H₄)₃ which are even more unstable and extremely air-sensitive (Fig. 1a).

For these reasons, the search for an alternative Ni(0) precursor which is stable under air has spurred chemists' minds for years, thus recognizing that such precatalyst would permit the development of facile and highly practical methodologies from the point of view of preparation time and reaction setup.

Indeed, the unique properties and reactivity of Ni(0)-olefin complexes are still of utmost importance and chemists have devoted great effort to manipulate such compounds under aerobic conditions, as exemplified by the development of other Ni(II) precatalysts (Fig. 1b) or paraffin capsules which permit the usage of Ni(COD)₂ in a benchtop setting.

However, there is still the need to provide a practical solution to the usage of an air-stable Ni(0) precursor.

Surprisingly, the inventors have developed the synthesis and investigated the catalytic activity of a unique 16-electron binary Ni(0)-olefin complex: Ni(^{F}stb)₃. The complex is characterized by its *p*-CF₃-stilbene derivatives as ligands, which render the Ni center remarkably stable under aerobic conditions (Fig. 1c).

Contrarily to all the reported 16- and 18-electron Ni(0)-olefin complexes, Ni(^{F}stb)₃ is stable under air for months without noticeable decomposition storing it in the freezer at -18 °C. The complex can be manipulated without the use of a glovebox or Schlenk and is highly modular, thus permitting ligand exchange with a variety of commonly employed ligands in Ni catalysis such as diamines, phosphines, N-heterocyclic carbenes (NHCs), etc, affording well-defined Ni(0)-L species. Moreover, its catalytic activity was benchmarked with that of Ni(COD)₂ and revealed to be an excellent precursor for a wide variety of different Ni-catalyzed reactions.

The present invention is therefore directed to Ni(R)₃-complex wherein Ni represents Ni(0) and R may be the same or different and represents a *trans-*stilbene of the Formula (I): 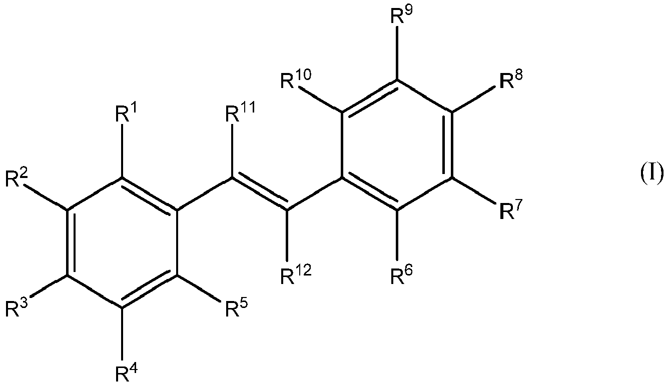
wherein R¹ to R¹⁰ may be the same or different and are selected from H, Cl, Br, F, CN, C₁ to C₈ alkyl or C₃ to C₆ cycloalkyl which alkyl or cycloalkyl may optionally be substituted by one or more halogens,
wherein R¹¹ to R¹² may be the same or different and are selected from H, C₁ to C₈ alkyl, C³ to C₆ cycloalkyl, -O-C₁ to C₈ alkyl or -O-C₃ to C₆ cycloalkyl,
with the proviso that at least one of R¹ to R¹² is not hydrogen.

In another embodiment of the inventive Ni(R)₃-complex, R is the same or different and in Formula (I), R³ and R⁸ are the same or different and are selected from C₁ to C₈ alkyl which alkyl or cycloalkyl may optionally be substituted by one or more halogens, and the others of R¹ to R¹⁰ are hydrogen, and R¹¹ to R¹² may be the same or different and are selected from H, C₁ to C₈ alkyl, C₃ to C₆ cycloalkyl, -O-C₁ to C₈ alkyl or -O-C₃ to C₆ cycloalkyl .

In a further embodiment of the inventive Ni(R)₃-complex, R is the same and in Formula (I), R³ and R⁸ are the same or different and are selected from C₁ to C₈ perfluoroalkyl and the others of R¹ to R¹⁰ are hydrogen, and R¹¹ to R¹² may be the same or different and are selected from H, C₁ to C₈ alkyl, C₃ to C₆ cycloalkyl, -O-C₁ to C₈ alkyl or -O-C₃ to C₆ cycloalkyl.

In yet another embodiment of the inventive Ni(R)₃-complex, R is the same and in Formula (I), R³ and R⁸ are each C₁ to C₈ perfluoroalkyl, preferably CF₃, and the others of R¹ to R¹⁰ and R¹¹ to R¹² are hydrogen.

In the invention, alkyl is intended to represent any alkyl group having one to eight carbon atoms including branched alkyl groups such as methyl, ethyl, propyl, isopropyl, butyl, iso-butyl, pentyl, iso-pentyl, hexyl, iso-hexyl, heptyl, iso-heptyl, octyl, iso-octyl.

In the invention, cycloalkyl is intended to represent any cycloalkyl group having three to six carbon atoms including alkyl groups such as cyclopropyl, cyclobutyl, cyclopentyl, cyclohexyl, and substituted alkyl rings.

Every alkyl or cycloalkyl group may be substituted by one or more halogens, particularly fluorine.

The present invention also refers to the use of the inventive air-stable Ni(R)₃ -complex as catalyst in organic synthesis, wherein
Ni represents Ni(0) and R may be the same or different and represents a *trans-*stilbene of the Formula (I): 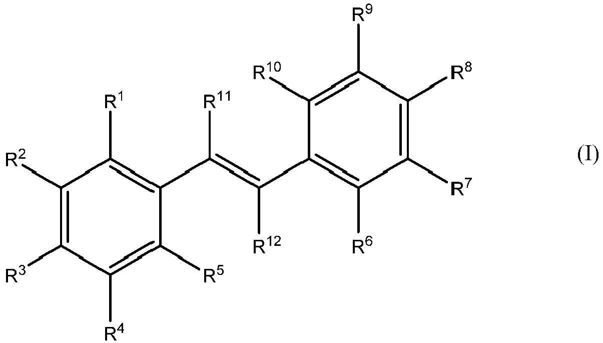
R¹ to R¹⁰ may be the same or different and are selected from H, Cl, Br, F, CN, C₁ to C₆ alkyl, C₃ to C₆ cycloalkyl which may optionally be substituted by one or more halogens,
R¹¹ to R¹² may be the same or different and are selected from H, C₁ to C₈ alkyl, C₃ to C₆ cycloalkyl, -O-C₁ to C₆ alkyl or -O-C₃ to C₆ cycloalkyl,
optionally with the proviso that at least one of R¹ to R¹² is not hydrogen.

### Catalytic properties

Having demonstrated the ability to exchange ligands with commonly employed ligands in Ni catalysis, the inventors set out to explore the catalytic properties of the inventive Ni(0)-olefin complex as a Ni(0) source in a variety of relevant organic transformations. To this end, the inventors benchmarked their catalyst in different Ni-catalyzed transformations as source of Ni(0), and compared the performance of the inventive Ni(0)-olefin complex versus Ni(COD)₂ and some Ni(II) precatalysts.

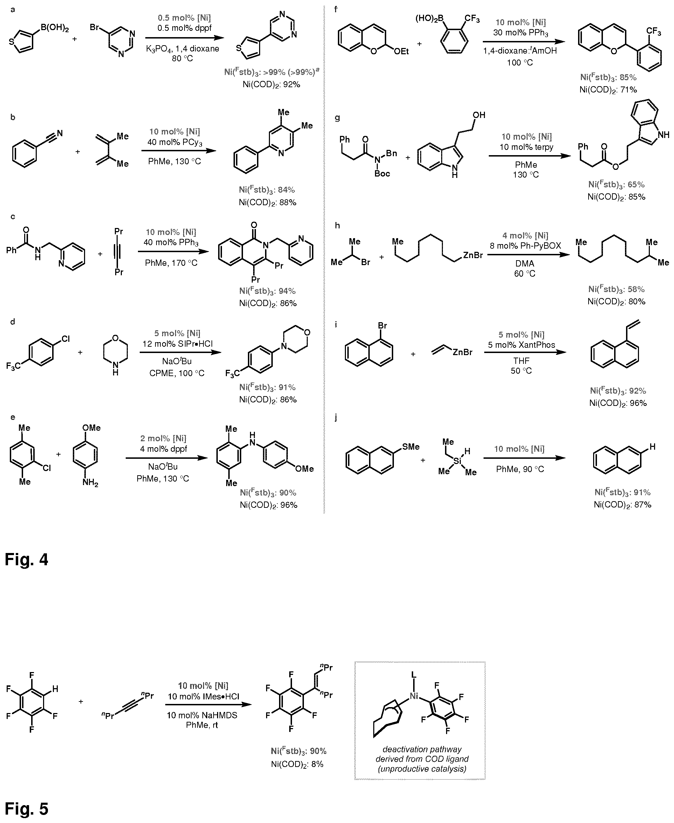

The inventors initially explored the feasibility to catalyze a Suzuki coupling due to its tremendous importance in modern synthesis. The use of the inventive Ni(0)-olefin complex as precatalyst permitted the coupling of an heteroaryl boronic acid and an heteroaryl bromide in excellent yield (Fig. 4a. >99%).

Another reaction of high interest is the oxidative cycloaddition between nitriles and dienes reported by Ogoshi. Despite the higher temperatures employed for the reaction (130 °C), the inventive Ni(0)-olefin complex proved stable and catalytically competent affording 84% yield of product (Fig. 4b).

C-H activation strategies based on Ni catalysis have recently emerged utilizing Ni(0) as a precatalyst source. As an example, Chatani demonstrated the synthesis of isoquinolones from simple amides and alkynes. Simple PPh₃ was reported to be the optimal ligand for such purposes; in this case, the inventive Ni(0)-olefin complex also proved an excellent candidate as a Ni(0) source affording excellent yields (Fig. 4c. 94%).

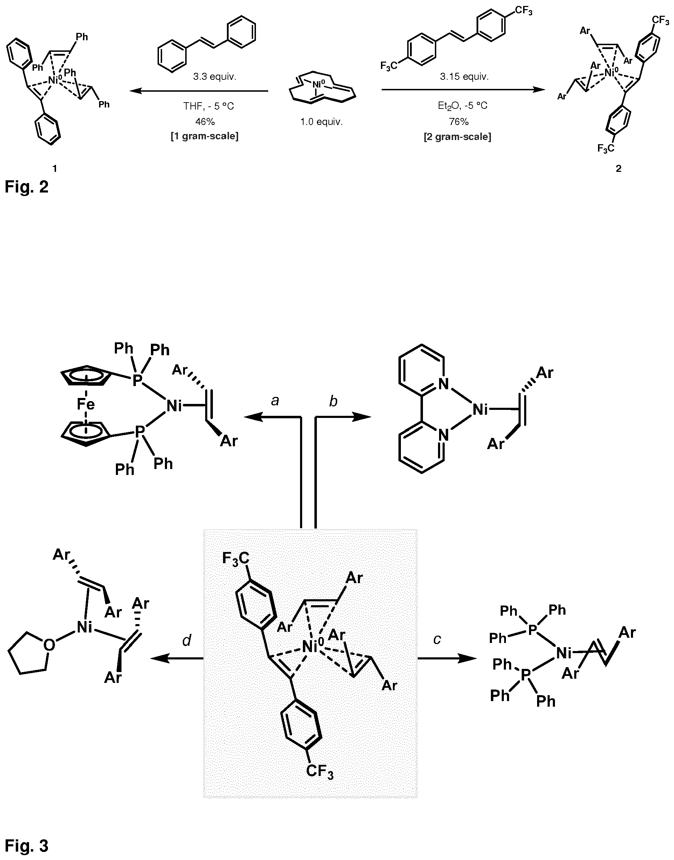

To further test the ability of the inventive Ni(0)-olefin complex as precatalyst, the inventors turned their attention to the formation of important C-N bonds. To this end, the inventors capitalized on reports for the amination of aryl halides with both aromatic and aliphatic amines. When SIPr is utilized as ligand, the inventive Ni(0)-olefin complex smoothly afforded the product in excellent yields (Fig. 4d. 91%). When aromatic amines were used instead, dppf was used as ligand and smooth conversion to the bis-aromatic amines was obtained (Fig. 4e. 90% yield). It is worth pointing out that in this latter example, slightly higher temperatures were required compared to the reported, presumably due to the high stability of the (dppf)Ni(0)(^{F}stb) intermediate (Fig. 3), thus requiring higher energy to promote the dissociation of the stilbene ligand compared to its Ni(COD)₂ analog.

Activation of acetals for arylation was recently reported by Doyle. Albeit the presence of protic solvents such as *^{t}*AmOH, the inventive Ni(0)-olefin complex proved to be an extremely good candidate, obtaining excellent yields of arylation (Fig. 4f. 85%). The ability of low-valent Ni species to activate amides through its C-N bond has been demonstrated to be a powerful disconnection for organic synthesis. The inventive Ni(0)-olefin complex bodes well in this context as highlighted by the high yields of ester formation from N-Me-Boc amides with tryptophol (Fig. 4g. 65%). The inventive Ni(0)-olefin complex is also amenable to excel as Ni(0) source in powerful alkyl-alkyl Negishi cross-couplings, as exemplified by the 58% yield of C-C bond obtained in Fig. 4h. It is worth mentioning that these last two reactions were successfully carried out using terpyridine and PyBOX derivatives as ligands, thus highlighting the facile conversion of the inventive Ni(0)-olefin complex to the active L-Ni(0) species with tridentate ligands.

A Negishi cross-coupling between an aryl bromide and a vinyl zinc reagent catalyzed by **2** could achieve similar yields than the corresponding Ni(0) precursors reported (92%, Fig. 4i). Ni(0)-olefin complexes have also been utilized as precursors for the generation of heterogeneous Ni(0) particles without the addition of ancillary ligands. In this context, the inventive Ni(0)-olefin complex proved to be an excellent candidate as shown in the reduction of thiomethylethers with silanes (91%, Fig. 4j).

Recently, the use of highly electron-donating ligands such as NHC in combination with Ni(COD)₂ has been the catalytic system of choice in the context of hydroarylation strategies via C-H activation. However, it has been noticed that this particular combination of catalyst and ligand leads to the formation of undesired Ni π-allyl complexes as a consequence of hydrometallation of the COD ligand. Structural evidences and reactivity studies have concluded that such species are preventing catalytic activity and turnover. The inventors envisaged that the inventive Ni(0)-olefin complex could avoid the detrimental pathways observed in certain C-H arylation strategies thus favoring productive catalysis. To test this hypothesis, the inventors examined the direct hydroarylation of alkynes using electron-deficient arenes. As reported, the use of Ni(COD)₂ in combination with an IMes afforded traces of hydroarylated product. On the other hand, the use of the inventive Ni(0)-olefin complex reacted smoothly at room temperature and a remarkable 90% yield of product was obtained (Fig. 5). This result highlights the fact that the inventive Ni(0)-olefin complex is competent as a Ni(0) precatalyst and in some instances, it could serve as a unique alternative when COD side reactions occur. It is important to mention that the use of the inventive Ni(0)-olefin complex did not require the use of the highly sensitive free carbene and the simple use of the parent HCl salt in combination with a base sufficed to achieve reactivity.

It is important to mention that in all examples where the inventive Ni(0)-olefin complex is the precatalyst, the reaction setup was performed in an open-air environment and in the bench, only restricting the use of glovebox to those ligands that are air-sensitive, such as PCy₃ (Fig 4b). Hence, the use of glovebox was dictated by the sensitivity of the optimal ligand for each particular case and in no case by the Ni-olefin precatalyst. Overall, these results highlight the competitiveness of Ni(^{F}stb)₃ (**2**) to act as an efficient Ni(0) source in a variety of catalytic contexts. Moreover, the good yields obtained when the inventive Ni(0)-olefin complex is operating highlight its modularity when ligands of different chelating nature or nucleophilicity are to be used.

The present invention is explained in more detail with reference to the Figures and Experimental Part.

The Figures illustrate:
- **Fig. 1.**: **a:** State-of-the-art binary Ni(0) olefin complexes for Ni catalysis.
**b:** Current strategies to circumvent the air-sensitivity issues related to Ni(0) species;
**c**: The present Invention as exemplified by Ni(^{F}stb)₃: an air-stable 16-electron Ni(0)-olefin complex.
- **Fig. 2**: Synthesis of complexes **1** and **2**:
Reaction conditions: all-*trans*-Ni(CDT) (1.0 equiv.), *trans*-stilbene or *trans*-(4-trifluoromethylphenyl)stilbene (3.30 and 3.15 equiv. respectively) at -5 °C in THF or Et₂O.
- **Fig 3**: Ligand exchange of complex **2** with different common ligands in catalysis:
a) **2** (1.0 equiv.), dppf (1.0 equiv.) in THF at 25 °C, quantitative;
b) **2** (1.0 equiv.), bipy (1.0 equiv.) in THF at 25 °C, quantitative;
c) **2** (1.0 equiv.), PPh₃ (2.0 equiv.) in THF at 25 °C, quantitative;
d) Slow crystallization of **2** in THF at -78 °C. Ar = *p*-CF₃-C₆H₄. Green: Ni; Black: C; Yellow: F; Orange: P; Deep orange: Fe; Red: O; Hydrogen atoms omitted for clarity.
- **Fig. 4**: Catalytic properties of **2** in a variety of Ni-catalyzed transformations.
**a.** Suzuki cross-coupling;
**b.** Cycloisomerization reaction;
**c.** C-H activation;
**d.** Buchwald-Hartwig C-N bond formation with alkylamines;
**e.** Buchwald-Hartwig C-N bond formation with arylamines;
**f.** C-O arylation of acetals;
**g.** Ester formation through C-N bond activation of amides;
**h.** Alkyl-alkyl cross-coupling;
**i.** Negishi cross-coupling;
**j.** C-SMe reduction with silanes.
- **Fig.** 5: Ni(^{F}stb)₃ avoids traditional COD side-reactions.

### General Experimental Notes

Unless otherwise stated, all manipulations were performed using Schlenk techniques under dry argon in heatgun-dried glassware. Ni(^{F}stb)₃ was stored in a screw cap vial under air in the freezer (-18 °C) and was weight out in air. Anhydrous solvents were distilled from appropriate drying agents and were transferred under Argon: THF, Et₂O (Mg/anthracene), CH₂Cl₂, CH₃CN (CaH₂), hexanes, toluene (Na/K), Et₃N, DMA, 1,4-dioxane (MS), CPME, NMP and *^{t}*AmOH were purchased in anhydrous grade and were stored over MS. Anhydrous K₃PO₄, NaO*^{t}*Bu and NaHMDS were stored in a Schlenk or in a glovebox. Flash column chromatography: Merck silica gel 60 (40-63 µm). MS (EI): Finnigan MAT 8200 (70 eV). Accurate mass determinations: MAT 95 (Finnigan). NMR spectra were recorded using a Bruker Avance VIII-300 or Bruker Avance III HD 400 MHz spectrometer. ¹H NMR spectra were referenced to the residual protons of the deuterated solvent used. ¹³C NMR spectra were referenced internally to the D-coupled ¹³C resonances of the NMR solvent. Chemical shifts (δ) are given in ppm, relative to TMS (tetramethylsilane), and coupling constants (*J*) are provided in Hz. ¹⁹F NMR spectra were referenced externally to the ¹⁹F resonances of CFCl₃. ³¹P NMR spectra were referenced externally to the ³¹P resonances of H₃PO₄.

### 1 Preparation of Ni(stb)₃ (1)

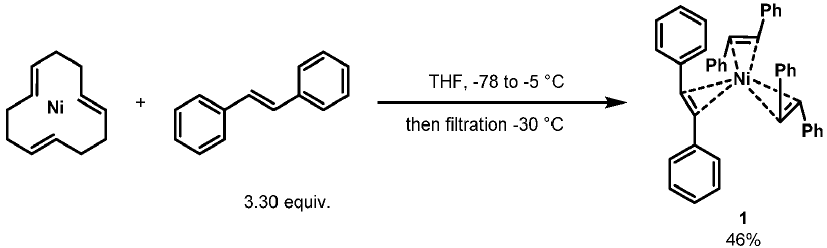

A Schlenk tube was charged with Ni(CDT) (CDT = 1,5,9-*trans,trans,trans-*cyclododecatriene) (794 mg, 3.60 mmol) *via* argon trousers and dissolved in THF (7 mL). The solution was filtered under argon into a Schlenk tube held at -78 °C. The filter cake was washed with 3 mL of THF. A separate Schlenk tube was charged with *trans*-stilbene (2.13 g, 11.87 mmol, 3.30 equiv.) and subjected to one cycle of vacuum/argon. The ligand was suspended in THF (10 mL) and transferred as a suspension to the first Schlenk tube, followed by one wash (2 mL THF) to ensure quantitative transfer. The reaction was stirred at -78 °C for 10 min and was then placed in a cooling bath at -5 °C and stirred at that temperature for 12 h. An argon frit was cooled to -30 °C and the reaction was transferred into the frit. The mixture was allowed to cool down for 1 min and was then filtered with positive pressure of argon. The solid on the frit was dried by passing a flow of argon through the frit. The solid was then transferred to a Schlenk tube and dried further under high vacuum at room temperature to give **1** as an air stable brown-red solid (1.07 g, 1.66 mmol, 46%).

### 2 Preparation of Ni(^{F}stb)₃ (2)

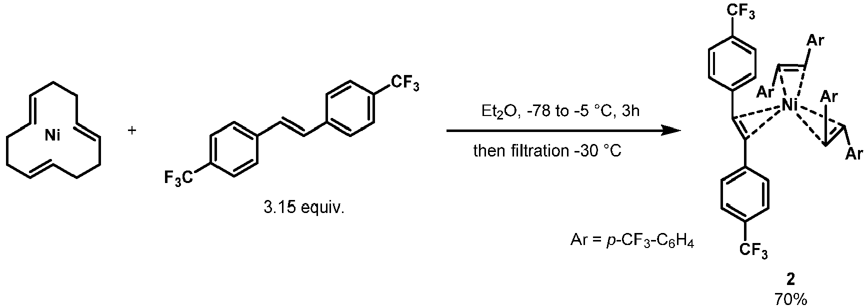

A Schlenk tube was charged with Ni(CDT) (CDT = 1,5,9-*trans,trans,trans-*cyclododecatriene) (610 mg, 2.76 mmol) *via* argon trousers and fresh Et₂O (10 mL) was added at -78 °C to suspend the starting material. A separate Schlenk tube was charged with *trans*-*p*CF₃-stilbene (2.28 g, 9.12 mmol, 3.15 equiv.) and subjected to one cycle of vacuum/argon. The ligand was suspended in Et₂O (10 mL) and transferred as a suspension to the first Schlenk tube, followed by several washings (3 + 2 + 2 mL) to ensure quantitative transfer. The reaction was placed in a cooling bath at -5 °C and stirred at that temperature for 3 h.

An argon frit was cooled to -30 °C and the reaction was transferred onto the frit. The reaction was allowed to cool down for 1 min and was then filtered with positive pressure of argon. The solid on the frit was washed with Et₂O (3 × 2 mL) and dried by passing a flow of argon through the frit. The solid was then transferred to a Schlenk tube and dried further under high vacuum at room temperature to give **2** as an air stable red solid (1.93 g, 1.92 mmol, 70%). The catalyst was stored under air in a freezer.

### 3 Synthesis of (E)-1,2-bis(4-(trifluoromethyl)phenyl)ethene (3)

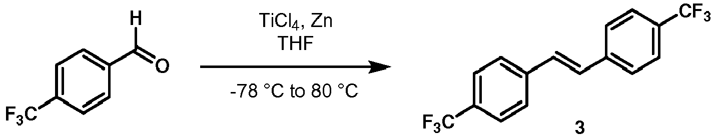

4-Trifluormethylbenzaldehyde (11.0 mL, 14.0 g, 80.5 mmol) was added to a 500 mL three necked round bottom flask equipped with a large stirring bar and reflux condenser. THF (200 mL) was added and the solution was cooled to -78 °C. TiCl₄ (11.00 mL, 19.03 g, 100.33 mmol, 1.25 equiv.) was added dropwise over 12 min. The reaction was taken out of the cooling bath, stirred for 10 min and then placed in the cooling bath again. Zn powder (13.0 g, 198 mmol, 2.5 equiv.) was added in several portions over 2 min. The reaction was allowed to reach room temperature and was then refluxed for 3h. After letting the reaction allow to cool to room temperature, water (300 mL) was added, followed by HCl (20 mL, 3M). The reaction was stirred for 5 min and transferred to a separation funnel. The aqueous layer was extracted with Et₂O (400 mL + 300 mL), the combined organic layers were washed with sat. aq. NaCl solution and dried over MgSO₄. The solvent was evaporated under reduced pressure and the residue was subjected to column chromatography (pure hexanes). The purified product was dried in high vacuum to give **3** as a colorless solid (8.44 g, 26.7 mmol, 66%).

### 4 Catalytic reactions

### 5-(Thiophen-3-yl)pyrimidine (4)

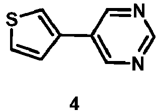

Ni(^{F}stb)₃ (2.0 mg, 0.002 mmol, 0.005 equiv.), 5-bromopyrimidine (64.5 mg, 0.406 mmol), thiophen-3-ylboronic acid (102.3 mg, 0.800 mmol, 2 equiv.), dppf (1.1 mg, 0.002 mmol, 0.005 equiv.) and anhydrous K₃PO₄ (135 mg, 0.64 mmol, 1.5 equiv.) were placed in a screw cap vial which was subsequently subjected to one cycle of vaccum/argon. 1,4-dioxane (1 mL) was added and the reaction was heated to 80 °C for 8 h. Water was added and the aqueous layer was extracted 3 times with 10 mL Et₂O. The combined organic layers were dried with MgSO₄ and evaporated under reduced pressure. The crude product was subjected to column chromatography (3:1 to 1:1; Hexanes:EtOAc) to yield **4** in analytically pure form as an white solid (66.7 mg, >99%). The same yield was obtained when a sample of complex **2** was used as precatalyst after storing it for >100 days in the freezer.

### 4,5-Dimethyl-2-phenylpyridine (5)

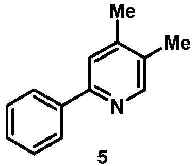

Ni(^{F}stb)₃ (50.4 mg, 0.05 mmol, 0.1 equiv.) was placed in a pressure tight Schlenk tube which was sealed and subjected to one cycle of vacuum/argon. The Schlenk tube was transferred to the glovebox PCy₃ (56.1 mg, 0.2 mmol, 0.4 equiv.) was added and the Schlenk was taken out of the glovebox again. Toluene (3 mL) was added followed by 2,3-dimethylbuta-1,3-diene (226.3 µL, 164.3 mg, 2 mmol, 4 equiv.) and benzonitrile (51.1 µL, 51.6 mg, 0.5 mmol). The Schlenk tube was sealed pressure tight and heated to 130 °C for 48 h. The solvent was removed under reduced pressure and the crude product was purified by column chromatography (9:1 to 5:1; Hexanes:EtOAc) to yield **5** as an yellow oil (76.7 mg, 0.419 mmol, 84%).

### 3,4-Dipropyl-2-(pyridin-2-ylmethyl)isoquinolin-1(2H)-one (6)

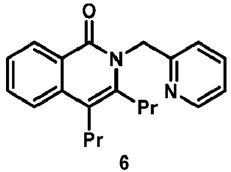

A 10-mL pressure-tight Schlenk tube was charged with *N*-(pyridin-2-ylmethyl)benzamide (106.1 mg, 0.50 mmol), PPh₃ (52.5 mg, 0.20 mmol, 0.4 equiv.), 4-octyne (0.22 mL, 1.50 mmol, 3.0 equiv.) and Ni(^{F}stb)₃ (50.4 mg, 0.05 mmol, 0.1 equiv.). Dry toluene (2 mL) was added and the reaction mixture was placed into a preheated oil bath at 170 °C and stirred for 20 h. After cooling to room temperature, the solvent was removed under reduced pressure. Purification of the crude residue *via* column chromatography (1:1; Hexanes:EtOAc) afforded pure **6** (151 mg, 0.47 mmol, 94%) as a yellowish oil.

### 4-(4-(Trifluoromethyl)phenyl)morpholine (7)

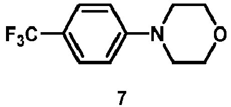

A 12 mL screw-cap vial was charged with Ni(^{F}stb)₃ (27.2 mg, 0.027 mmol, 0.05 equiv.), SIPr·HCl (26.8 mg, 0.063 mmol, 0.116 equiv.) and dry CPME (1.5 mL). 4-chlorobenzotrifluoride (72 µL, 0.540 mmol, 1.00 equiv.) and morpholine (57 µL, 0.648 mmol, 1.20 equiv.) were added to the solution. While stirring the solution for 15 min at room temperature the mixture became orange-yellowish. NaO*t*Bu (2 M in THF, 543 µL, 1.080 mmol, 2.00 equiv.) was then added and the brown reaction mixture was stirred for 4 h in a preheated oil bath at 100 °C. After cooling to room temperature the solvent was removed under reduced pressure. Column chromatography of the crude residue (9:1; Hexanes:EtOAc) afforded **7** (114 mg, 0.493 mmol, 91% yield) as a colorless solid.

### N-(4-Methoxyphenyl)-2,5-dimethylaniline (8)

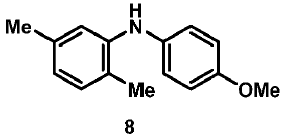

A 12 mL screw-cap vial was charged with Ni(^{F}stb)₃ (20.1 mg, 0.02 mmol, 0.02 equiv.), dppf (22.2 mg, 0.04 mmol, 0.04 equiv.) and anhydrous sodium *t-*butoxide (134.5 mg, 1.40 mmol, 1.40 equiv.). Toluene (2 mL) was added followed by 2-chloro-*p*-xylol (0.134 mL, 1.00 mmol, 1.00 equiv.) and *p*-anisidine (147.8 mg, 1.20 mmol, 1.20 equiv.). Additional toluene (2 mL) was added and the vial was set into a preheated oil bath at 130 °C and stirred for 48 h. After cooling to room temperature the reaction mixture was diluted with EtOAc and water was added and the layers were separated. The aqueous layer was extracted with EtOAc and the combined organic layers were dried over MgSO₄. The solvent was removed under reduced pressure, and the crude residue was purified *via* column chromatography (gradient: 50:1 to 20:1; Hexanes:EtOAc) to afford **8** as an orange oil (205.1 mg, 0.90 mmol, 90% yield).

### 2-(2-(Trifluoromethyl)phenyl)-2H-chromene (9)

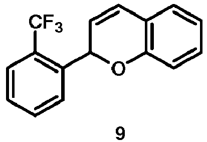

A 12 mL screw cap vial was charged with Ni(^{F}stb)₃ (58.6 mg, 0.05 mmol, 0.09 equiv.) and PPh₃ (39.3 mg, 0.15 mmol, 0.27 equiv.). 1,4 dioxane (1 ml) was added and the solution was stirred for 5 min. A 50 mL Schlenk tube was charged with 2-ethoxy-2*H*-chromene (98.9 mg, 0.56 mmol), (2-(trifluoromethyl)phenyl)boronic acid (189.9 mg, 1.00 mmol, 1.78 equiv.), dioxane (23 mL) and *t*-AmOH (2 mL). The catalyst + ligand solution was transferred two the second Schlenk tube and the reaction was placed in a preheated oil bath at 100 °C for 40 min. The reaction as allowed to cool down and the solvents were evaporated under reduced pressure. The residue was subjected to column chromatography (pure hexanes) to give pure **9** as a colorless oil (131.9 mg, 0.56 mmol, 85%).

### 2-(1H-Indol-3-yl)ethyl 3-phenylpropanoate (10)

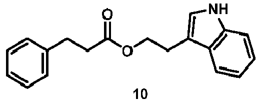

A 12 mL screw-cap vial was charged with Ni(^{F}stb)₃ (20.1 mg, 0.02 mmol, 0.1 equiv.), terpyridine (4.7 mg, 0.02 mmol, 0.1 equiv.), *tert*-butyl benzyl(3-phenylpropanoyl)carbamate (67.9 mg, 0.20 mmol, 1.00 equiv.) and tryptophol (40.3 mg, 0.25 mmol, 1.25 equiv.). Toluene (0.2 mL) was added and the vial was set into a preheated oil bath at 130 °C. After stirring for 23 h the solution was allowed to cool to room temperature and the contents were transferred with EtOAc and hexanes into a round-bottomed flask. The solvent was removed under reduced pressure and the crude residue was purified *via* column chromatography (7:1; Hexanes/EtOAc) to afford **10** as an orange oil (38.5 mg, 0.13 mmol, 65% yield).

### 2-Methylundecane (11)

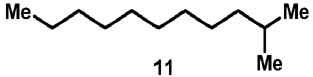

A 12 mL screw-cap vial was charged with Ni(^{F}stb)₃ (10.0 mg, 0.010 mmol, 0.04 equiv.) and 2,6-bis((*R*)-4-phenyl-4,5-dihydrooxazol-2-yl)pyridine (7.4 mg, 0.020 mmol, 0.08 equiv.). DMA (0.4 mL) was added under argon, the deep-blue solution was stirred for 10 min at room temperature and tetradecane (internal standard for GC analysis, 20 µL, 0.077 mmol) was added. The mixture was stirred for further 10 min. at room temperature and a solution of n-nonylzinc bromide (0.85 M in DMA, 0.47 mL, 0.400 mmol, 1.57 equiv.) and *i*-propyl bromide (24 µL, 0.256 mmol, 1.00 equiv.) were added. After stirring the reaction mixture for 20 hours at 60 °C a 58% yield of **11** was determined by GC-FID analysis.

### 1-Vinylnaphthalene (12)

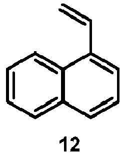

A screw cap vial was charged with Ni(^{F}stb)₃ (10.1 mg, 0.01 mmol, 0.05 equiv.) once cycle of vacuum/ argon was performed and the vial was transferred to a glovebox. Xantphos (5.8 mg, 0.01 mmol, 0.05 equiv.) was added and the vial was removed from the glovebox. THF (150 µL), 1-bromonaphthalene (28.0 µL, 41.4 mg, 0.2 mmol) and vinylzincbromide (1M in THF/NMP, 350 µL, 1.75 equiv.) were added. The reaction was heated to 50 °C for 5 h and was subsequently diluted with EtOAc. Mesitylene (25 µL 21.6 mg) was added as internal standard and a 92% yield of **12** was determined *via* GC-FID analysis.

### Naphtalene (13)

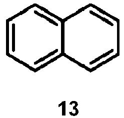

A 12 mL screw-cap vial was charged with 2-(methylthio)naphthalene (87.2 mg, 0.50 mmol, 1.0 equiv.) and Ni(^{F}stb)₃ (50.4 mg, 0.05 mmol, 0.1 equiv.). *n-*Dodecane (internal standard for GC analysis, 20 µL, 0.09 mmol), EtMe₂SiH (0.13 mL, 0.98 mmol, 2.0 equiv.) and toluene (2 mL) were added. The vial was set into a preheated oil bath at 90 °C and kept stirring for 14 hours. After cooling to room temperature the mixture was diluted with EtOAc (4 mL) and a 91% yield of **13** was determined *via* GC-FID analysis.

### 1,2,3,4,5-Pentafluoro-6-(oct-4-en-4-yl)benzene (14)

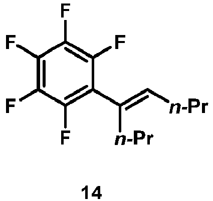

A screw cap vial was charged with Ni(^{F}stb)₃ (20.1 mg, 0.02 mmol, 0.1 equiv.) IMes•HCl (6.8 mg, 0.02 mmol, 0.1 equiv.) and anhydrous NaHMDS (3.6 mg, 0.02 mmol, 0.1 equiv.). Toluene (1.5 mL) was added and the mixture was stirred for 5 min. 1,2,3,4,5-pentafluorobenzene (22.2 µL, 33.6 mg, 0.2 mmol) and 4-octin (44.0 µL, 33.1 mg, 0.3 mmol, 1.5 equiv.) were added and toluene (0.5 mL) was used to wash the substrates down. The reaction as stirred at rt for 3h and was quenched by addition of CH₂Cl₂. The mixture was filtered over a plug of silica and evaporated to dryness. α,α,α-Trifluortoluol (24.6 µL, 29.2 mg, 0.2 mmol, 1.0 equiv.) was added as internal standard and the yield (90%) was determined by ¹⁹F NMR.

As stated above, a long-standing problem in the area of Ni catalysis has been solved by providing the inventive complex as a Ni(0) precatalyst which mimics the remarkable reactivity of Ni(COD)₂ but has the advantages of being robust, air-stable and easy to handle in open-flask conditions. Herein, the inventors reported the synthesis and characterization of a binary Ni(0)-olefin complex that fulfills all these requirements and permits Ni catalysis without the use of complex Schlenk techniques or gloveboxes. The inventive Ni(0)-olefin complex Ni(R)₃ is the first air-stable binary Ni(0)-olefin complex which has remarkable stability under air and benefits from a high reactivity in solution due to its 16-electron configuration. Its catalytic abilities have been benchmarked with those of Ni(COD)₂ and the inventors have shown that Ni(R)₃ is an excellent precatalyst in a range of Ni-catalyzed transformations. Differently than the common air-stable precursors based on Ni(II) complexes, Ni(R)₃ is characterized by its intrinsic ability to deliver Ni(0) species in solution and afford discrete and well-defined Ni(0)-Ligand complexes. The great performance of Ni(R)₃ as Ni(0) precatalyst is envisaged to rapidly expand to all areas of Ni catalysis thus permitting facile setups and accelerating the discovery of new reactivity.

## Claims

1. An air-stable Ni(R)₃-complex wherein Ni represents Ni(0) and R may be the same or different and represents a *trans*-stilbene of the Formula (I): 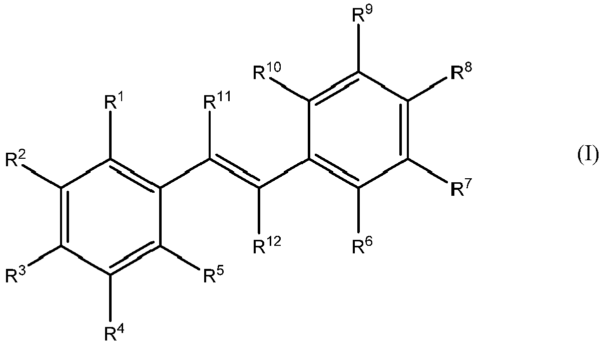
wherein R¹ to R¹⁰ may be the same or different and are selected from H, Cl, Br, F, CN, C₁ to C₆ alkyl or C₃ to C₆ cycloalkyl which alkyl or cycloalkyl may optionally be substituted by one or more halogens,
wherein R¹¹ to R¹² may be the same or different and are selected from H, C₁ to C₆ alkyl, C₃ to C₆ cycloalkyl, -O-C₁ to C₆ alkyl, or -O-C₃ to C₆ cycloalkyl with the proviso that at least one of R¹ to R¹² is not hydrogen.

2. An air-stable Ni(R)₃-complex according to claim 1,
wherein Ni represents Ni(0),
wherein R is the same or different and represents a trans-stilbene of the Formula (I):
wherein in Formula (I), R³ and R⁸ are the same or different and are selected from C₁ to C₈ alkyl which may optionally be substituted by one or more halogens, and the others of R¹ to R¹⁰ are hydrogen, and R¹¹ to R¹² may be the same or different and are selected from H, C₁ to C₆ alkyl, -O-C₁ to C₆ alkyl, C₃ to C₆ cycloalkyl or -O-C₃ to C₆ cycloalkyl.

3. An air-stable Ni(R)₃-complex according to claim 1,
wherein Ni represents Ni(0),
wherein R is the same or different and represents a trans-stilbene of the Formula (I):
wherein in Formula (I), R³ and R⁸ are the same or different and are selected from C₁ to C₈ perfluoro alkyl and the others of R¹ to R¹⁰ are hydrogen, and R¹¹ to R¹² may be the same or different and are selected from H, C₁ to C₆ alkyl, O-C₁ to C₆ alkyl, C₃ to C₆ cyclolkyl or -O-C₃ to C₆ cycloalkyl.

4. An air-stable Ni(R)₃-complex according to claim 1,
wherein Ni represents Ni(0),
wherein R is the same and represents a *trans*-stilbene of the Formula (I):
wherein in Formula (I), R³ and R⁸ are each C₁ to C₈ perfluoro alkyl and the others of R¹ to R¹⁰ and R¹¹ to R¹² are hydrogen.

5. An air-stable Ni(R)₃-complex according to claim 1,
wherein Ni represents Ni(0),
wherein R is the same and represents a trans-stilbene of the Formula (I):
wherein in Formula (I), R³ and R⁸ are each -CF₃, and the others of R¹ to R¹⁰ and R¹¹ to R¹² are hydrogen.

6. Use of an air-stable Ni(R)₃-complex according to claim 1 as catalyst or precatalyst in organic synthesis, wherein:
Ni represents Ni(0) and R may be the same or different and represents a *trans*-stilbene of the Formula (I): 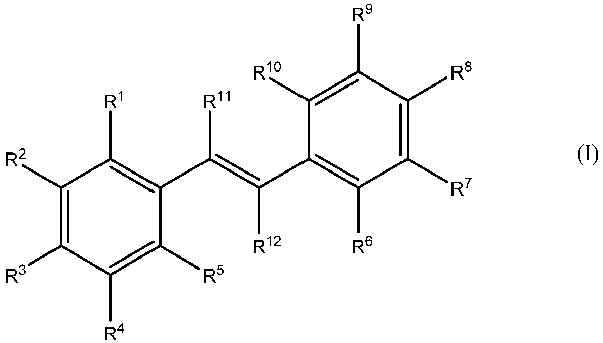
R¹ to R¹⁰ may be the same or different and are selected from H, Cl, Br, F, CN, C₁ to C₆ alkyl or C₃ to C₆ cycloalkyl which may optionally be substituted by one or more halogens,
R¹¹ to R¹² may be the same or different and are selected from H, -O-C₁ to C₆ alkyl, C₁ to C₆ alkyl or C₃ to C₆ cycloalkyl,
optionally with the proviso that at least one of R¹ to R¹² is not hydrogen.

7. Use of an air-stable Ni(R)₃-complex according to any one of claims 2 to 5 as catalyst in organic synthesis.
